(19) Europäisches Patentamt
European Patent Office
Office européen des brevets

(11) Veröffentlichungsnummer: **0 054 634**
**A1**

# (12) EUROPÄISCHE PATENTANMELDUNG

(21) Anmeldenummer: **81107967.2**

(22) Anmeldetag: **06.10.81**

(51) Int. Cl.³: **C 07 C 21/24**, C 07 C 25/00, C 07 C 17/38

(30) Priorität: **23.12.80 DE 3048627**

(43) Veröffentlichungstag der Anmeldung: **30.06.82**
**Patentblatt 82/26**

(84) Benannte Vertragsstaaten: **BE CH DE FR GB IT LI NL**

(71) Anmelder: **DYNAMIT NOBEL AKTIENGESELLSCHAFT,**
**Patentabteilung Postfach 1209, D-5210 Troisdorf, Bez. Köln (DE)**

(72) Erfinder: **Herrmann, Hans-Joachim, Dr.,**
**Eschenmattstrasse 5, CH-4313 Möhlin/Schweiz (CH)**
Erfinder: **Meyer, Günther, Dr., Augustinusstrasse 6,**
**D-5210 Troisdorf (DE)**
Erfinder: **Steffen, Klaus-Dieter, Dr., Röckelstrasse 36,**
**D-5202 Hennef-Geisbach (DE)**

(54) **Verfahren zur Reindarstellung von seitenkettenchlorierten Alkylaromaten durch Rektifikation.**

(57) Reine seitenkettenchlorierte Alkylaromaten werden durch Destillation von Chlorierungsgemischen der Alkylaromaten mit Chlorgehalten zwischen 4 und 65 Gew.-% Chlor in der Seitenkette oder deren im Kern chlorierten Derivaten durch fraktionierte Destillation bei Sumpftemperaturen von 100 bis 180 °C und vermindertem Druck von 1 bis 120 mbar in Füllkörper-Kolonnen aus Glas oder Keramik hergestellt.

EP 0 054 634 A1

Troisdorf, den 19. Dez. 1980
OZ: 80 107 (3085)

DYNAMIT NOBEL AKTIENGESELLSCHAFT
Troisdorf, Bez. Köln

# Verfahren zur Reindarstellung von seitenkettenchlorierten Alkylaromaten durch Rektifikation

Die Chlorierung von Xylolen und Toluolen unter Lichtkatalyse verläuft nach einem radikalischen Mechanismus, wodurch die Selektivität gering ist und Gemische von chlorierten Verbindungen erhalten werden. Deren Bestandteile können u.a. die Stoffe I bis XXXII sein:

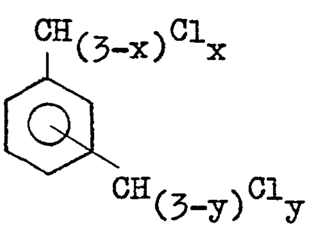

Xylole

| x | 0 | 1 | 2 | 3 | 0 | 2 | 3 | 3 |
|---|---|---|---|---|---|---|---|---|
| y | 1 | 1 | 1 | 1 | 2 | 2 | 2 | 3 |
| o- | I | II | III | IV | V | VI | VII | - |
| m- | VIII | IX | X | XI | XII | XIII | XIV | XV |
| p- | XVI | XVII | XVIII | XIX | XX | XXI | XXII | XXIII |

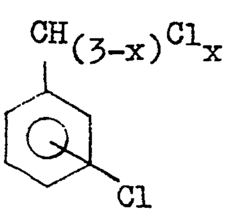

Chlortoluole

| x | 1 | 2 | 3 |
|---|---|---|---|
| o- | XXIV | XXV | XXVI |
| m- | XXVII | XXVIII | XXIX |
| p- | XXX | XXXI | XXXII |

Mit Ausnahme des sechsfach in der Seitenkette chlorierten o-Xylols, das aus räumlichen Gründen nicht entsteht, und der unwahrscheinlichen an einer Methylgruppe dreifach, an der anderen nicht chlorierten Xylole, bilden jeweils drei bis fünf der denkbaren Stoffe das typische Chlorierungsgemisch eines Xylols oder Chlortoluols. Lediglich bei Perchlorierung entstehen VII, XV, XXIII, XXVI, XXIX und XXXII als annähernd reine Stoffe.

Bedarf besteht für die weniger weit chlorierten Verbindungen als Zwischenprodukte. Die Trennung dieser Stoffe aus den Chlorierungsgemischen ist aber schwierig, zumal die Stoffe hierbei extrem korrosiv und thermisch labil sind und zu Kondensationsreaktionen ( vgl. GB-PS 1 410 474 ) neigen.

Nach dem Stand der Technik werden verschiedene Maßnahmen ausgeführt, ohne daß diese bei anderen Stoffmischungen erfolgreichen Mittel zu reinen Stoffen oder wesentlich erhöhter Anreicherung führen:

Zusätze von Drittstoffen bei der Chlorierung bewirken nur geringe Erhöhungen der Selektivität.

Kristallisationsverfahren erfordern mehrfaches Kristallisieren und ergeben geringe Ausbeuten bei wenig befriedigender Reinheit.

Destillative Trennverfahren sind schwierig und ergeben häufig Produktgemische: Nach der GB-PS 1 410 474 ist $\alpha$-Chlor-p-Xylol (XVI) in Füllkörper-Kolonnen bei Gegenwart von z.B. Aminen von einem Gemisch mit Gehalten von $\alpha,\alpha'$-Dichlor-p-Xylol (XVII) trennbar. Nach der bekanntgemachten japanischen Patentanmeldung 54/125 628 (1979) ( Banyu Pharmaceutical Co. ) ist $\alpha,\alpha,\alpha'$-Trichlor-p-Xylol (XVIII), durch Destillation gewinnbar. Nach BE-PS 590 772 erhält man $\alpha,\alpha'$-Dichlor-p-Xylol (XVII) aus Chlorierungsgemischen nur als angereichertes Rückstandsprodukt.

Bei allen Destillationsverfahren gelingt es mit tragbarem technischen Aufwand nicht, Produkte ausreichender Reinheit zu erzeugen. Lediglich Gemische mit weitem Siedebereich und mehr oder minder hoher Anreicherung der gewünschten Komponente gegenüber dem Chlorierungsgemisch sind erhältlich. Soweit Drittstoffe zugesetzt wurden, sind diese wieder zu entfernen. Mit erforderlicher Reinheit ist kein einzelner Stoff gewinnbar. Allgemein ist daher ein anschließender Kristallisationsprozeß zur Reinigung erforderlich.
Insbesondere ist die destillative Gewinnung der höherchlorierten Verbindungen, wie VI, XIII oder XXI, die nur noch geringe Flüchtigkeitsdifferenzen aufweisen, bisher nicht beschrieben.

Es bestand daher die Aufgabe, mit vertretbarem Aufwand die Einzelkomponenten von Gemischen seitenkettenchlorierter Alkylaromaten in guten Ausbeuten als Produkte ausreichender Reinheit abzutrennen und dabei anfallende Nebenprodukte einer sinnvollen Verwertung zuzuführen.

Gegenstand der Erfindung ist daher ein Verfahren zur Reindarstellung seitenkettenchlorierter Alkylaromaten durch Destillation, welches dadurch gekennzeichnet ist, daß Gemische beliebiger Zusammensetzung mit Chlorgehalten zwischen 4 und 65 Gew.-% Chlor in der Seitenketten, hergestellt durch Seitenkettenchlorierung von Alkylaromaten oder deren im Kern chlorierten Derivaten, durch fraktionierte Destillation bei Sumpftemperaturen von 100 bis 180°C und vermindertem Druck von 1 bis 120 mbar, bevorzugt von 6 bis 30 mbar, in die einzelnen Komponenten aufgetrennt werden, wobei Füllkörper-Kolonnen mit aus Glas oder keramischem Material bestehenden Kolonnenpackungen Verwendung finden, deren Druckverlust pro theortetischer Trennstufe weniger als ein mbar im gesamten Betriebsbereich beträgt und Spuren von Schwermetallen ausgeschlossen sind.

Als Alkylaromaten werden besondere die einkernigen Aromaten mit Seitengruppen aus besonders den Methylgruppen verstanden sowie deren Kernchlorierungsprodukte mit geringen Chlorgehalten. Besonders werden die Xylole und Toluol, jedoch auch das im Kern mit einem, gegebenenfalls mit zwei Chloratomen substituierte Toluol als Alkylaromaten verstanden.

Der jeweilige Chlorierungsgrad von 4 bis 65 Gew.-% des in der Seitenkette chlorierten Alkylaromaten hat sich nach dem Zielprodukt zu richten, derart, daß möglichst ein hoher Anteil des Zielprodukts im Einsatzstoff vorhanden ist, jedoch kann als Einsatzstoff auch der Vorlauf oder Nachlauf einer vorangehenden Rektifikation gewählt werden.

Bei Berechnung des Chlorierungsgrades der Seitenkette bleibt Chlor als Substituent des aromatischen Kerns, soweit vorhanden, z.B. im Gemisch des seitenkettenchlorierten p-Chlortoluols, unberücksichtigt.

Die Kolonnenpackungen sollen streng aus Materialien bestehen, die Schwermetalle nicht durch Korrosionswirkung der chlorierten Alkylaromaten abgeben. Als Material ist dann Tantal, insbesondere jedoch keramische Materialien erforderlich.

Dem entspricht, daß Schwermetalle, auch in Spuren generell ausgeschlossen sein sollen, so daß in zu destillierenden Gemischen Schwermetalle oder deren Verbindungen nur in Mengen von kleiner 0,5 ppm, berechnet als Metall, vorkommen sollen.

Das keramische Material der Kolonnenpackungen darf darüber hinaus nicht solche Metalle oder Metalloxide abgeben, die, wie besonders Aluminium zur Bildung von Lewis-Säuren führen.

Die in den Seitenketten chlorierten Alkylaromaten neigen nämlich ungleich stärker als andere chlorierte Kohlenwasserstoffe zur Bildung kondensierter Aromaten aus zwei oder mehr Molekülen unter Abspaltung von Chlorwasserstoff, wodurch bisher hohe Ausbeuteverluste auftraten, die Destillation durch " Gelbildung " erschwert war und das Vakuum durch den gasförmigen Chlorwasserstoff zusammenbrach.

Umgekehrt ist ein unvermindert aufrecht haltbares Vakuum das untrügliche Anzeichen, daß Chlorwasserstoff oder andere Zersetzungsprodukte nicht auftreten und weder die Kondensation durch Lewis-Säuren noch die katalytische Zersetzung durch Spuren von Schwermetallen oder deren Verbindungen erfolgen.

Soweit bekannt, ist bei der Destillation der seitenkettenchlorierten Alkylaromaten erstmals im Rahmen der vorliegenden Erfindung die Kondensation und katalytische Zersetzung der Produkte vermeidbar und damit auch erstmals der verminderte Druck von 1 bis 120 mbar bei der Vakuum-Rektifikation in technisch brauchbarer Weise aufrechterhaltbar.

Überraschend wurde gefunden, daß nicht nur Glas, das solche Schwermetalle und Lewis-Säuren bildenden Metalle nicht enthält, sondern auch keramische Materialien, in denen Schwermetalle und gegebenenfalls Aluminium durchaus enthalten sind, als Material der Kolonnenpackungen Verwendung finden können.

Offenbar ist es lediglich erforderlich, ein keramisches Material zu verwenden, dessen oxidische bzw. silikatische Bestandteile durch Glühen, Sintern oder ähnliche Behandlungen bei hohen Temperaturen eine Struktur z.B. in Art der Sinterkeramik erhalten hatten, aus der während der Rektifikation Bestandteile mit Zersetzungswirkung auf Ausgangsstoffe oder Produkte nicht freigesetzt werden können.

Als besonders geeignet erwiesen sich keramische Materialien aus 10 - 40 Gew.-% $ZrO_2$ und 90 - 60 Gew.-% $SiO_2$, vorzugsweise 15 bis 22 Gew.-% $ZrO_2$ und 80 bis 60 Gew.-% $SiO_2$, welche analytisch $Fe_2O_3$, Ni und Mn jeweils in Mengen von etwa 0,01 % aufweisen können und gegebenenfalls auch kleinere oder größere Anteile $Al_2O_3$ enthalten können. Es überrascht, daß $ZrO_2$ als Oxid oder gegebenenfalls als Silikat in geglühter bzw. gesinterter Form nicht zur Bildung von Lewis-Säuren Anlaß gibt. Desgleichen sind Gehalte von Schwermetallen als Bestandteil der oxidischen bzw. silikatischen Keramik unschädlich, anders als Spuren von Schwermetallen in Verunreinigungen der Oberfläche, die sich bei der ersten Benutzung bemerkbar machen und durch die erste Benutzung oder gegebenenfalls eine Behandlung mit Salzsäure entfernt werden.

Zur Erzielung des geringen Druckverlusts von weniger als ein mbar pro theoretischer Trennstufe im gesamten Betriebsbereich sind weiter Kolonnenpackungen sehr bevorzugt, die eine geordnete, sich stetig wiederholende Hohlstruktur in allen drei Raumrichtungen aufweisen.
In diesen " geordneten " Kolonnenpackungen soll die Raumerfüllung nur 10 - 30 %, bevorzugt 10 - 20 %, betragen.
Wichtig ist, daß die Anordnung der keramischen Strukturelemente in der Kolonnenpackung keine senkrechten Hohlkanäle im Inneren aufweist und an den Kolonnenmantel innen dicht anschließt oder abgedichtet wird.
Zweckmäßig besteht daher die Kolonnenpackung aus mehreren übereinander angeordneten bevorzugt gleichartigen Teilpackungen, in den die Strukturelemente zu einem einzigen mit Hohlräumen durchsetzten verbunden sind. Bei kleineren Kolonnen haben die Teilpackungen bevorzugt die äußere Gestalt von Zylindern oder Zylindersegmente oder gegebenenfalls Scheiben deren Durchmesser zur Höhe etwa 2 : 1 bis 1 : 4, bevorzugt 1 : 1,5 bis 1 : 3,5 beträgt. Bei größeren

Kolonnen kann die äußere Gestalt neben den genannten auch die Form von Lagen, Streifen o.ä. haben, die eng aneinander liegend angeordnet werden.
Stets besitzen die Packungen an den Berührungsflächen mit der Kolonnenwand einen oder mehrere Dichtungsstreifen, aus z.B. Asbestschnur oder -streifen oder aus Ringen oder Streifen aus Fluorpolymeren, die gegen chlorierte Alkylaromaten inert sind und horizontal so angeordnet sind , daß aufsteigende Dämpfe des Destillats und der Rücklauf von der Kolonnenwand in das Innere der Packung geleitet werden.
Sehr bevorzugt sind keramische Kolonnenpackungen, mit der äußeren Gestalt von besonders Zylindern oder gegebenenenfalls Scheiben oder Streifen, in deren geordneten stetiger Struktur im Inneren Stege, Streifen, Bänder, Stäbe oder Wendel im Winkel von 20 bis $70^{o}$ gegen die Vertikale so parallel in Schichten zueinander angeordnet sind, daß sich Kanäle gleichen Neigungswinkels $\alpha$ zur Vertikalen ergeben, die sich zu vertikalen Schichtebenen ordnen, auf die in horizontaler Sicht eine Schichtebenene mit Kanälen gleichen Neigungswinkels $\alpha$ in der anderen Raumrichtung folgt.
Bei den Stegen Streifen o.ä. liegen also zwei aufeinander folgende Schichten nach Art eines Gitters aufeinander , so daß in Sicht senkrecht auf die Schichtebenen, auf die parallelen Kanäle einer Schicht die parallelen Kanäle der nächsten Schicht im Winkel von 2 x $\alpha$ zu den Kanälen der ersten Schicht folgen und jeder Kanal einer Schicht regelmäßig verbindende Öffnungen zu Kanälen der nächsten Schicht besitzt und die Stege, Streifen o.ä. an allen Berührungspunkten mit Stegen, Streifen o.ä. der nächsten Schicht eine Verbindung aufweist.
Der aufsteigende Gasstrom ist also gezwungen, im Winkel zur Vertikalen aufzusteigen und wird durch die verbindenden Öffnungen auf die nächsten Kanäle verteilt, wobei das

rückfließende Kondensat gleichartig verteilt wird und gleichmäßig über die gesamte innere Oberfläche aller Strukturelemente der Kolonnenpackung verteilt wird und mit dem Gasstrom eine maximale Berührungsfläche bildet. Das Verteilungsgleichgewicht der Stoffe kann sich so, spezifisch je nach der Höhenlage in der Kolonne, sehr schnell einstellen.
Zusätzlich kann die Oberfläche der Packung weiter erhöht werden, dadurch, daß die Stege, Streifen o.ä. ein Profil aufweisen, z.B. dreieckige, rinnenförmige, kreuzförmige Querschnitte darstellen.
Weiter können die keramischen Materialien der Stege, Streifen o.ä. nach dem Brennen einen haftenden Auftrag von Keramikkörnungen gleicher oder verschiedener Art erhalten, dann zu den Packungen angeordnet werden und erneut gesintert oder gebrannt werden, wodurch die Keramikkörnungen an den Berührungsstellen der Stege, Streifen o.ä. schmelzen oder erweichen und nach dem Erkalten eine bleibende feste Verbidnung der Stege, Streifen o.ä. an allen Berührungsstellen bewirken, so daß jedes Packungselement eine einzige mit Kanälen durchzogene Hohlstruktur darstellt, die einfach gehandhabt werden kann.
Zusätzlich erhöht die festgesinterte Keramikkörnung wesentlich die innere Oberfläche der Hohlstruktur.

Geordnete, sich wiederholende Strukturen der Kolonnenpackung sind jedoch auch mit sich kreuzenden Glasstab-Profilen, Glasfaserbündeln o.ä. möglich, wobei ebenfalls Verbindungen zwischen den Profilen z.B. durch Beschichtung mit Silanen o.ä. wie auch vergrößerte innere Oberflächen durch Ätzen, Aufschmelzen von inertem Glasstaub, Aufbringen von Silanen o.ä. bevorzugt sind.
Gleichgültig, welche innere Struktur die Kolonnenpackungen besitzen, ist es verfahrensgemäß ganz entschieden bevorzugt, daß die gesamte Kolonnenpackung oder zweckmäßig

jede Teilpackung eine geordnete sich stetig in allen Raumrichtungen wiederholende Hohlstruktur aufweisen. Sehr bevorzugt ist weiter eine Raumerfüllung von nur 10 - 30 % der Kolonnenpackung durch das feste Material der Stege, Streifen o.a., so daß 90 bis 70 % freier Raum verbleibt.

Die Teilpackungen werden zweckmäßig so aufeinander gelegt, daß keiner die Kanäle der einen Packung die Kanäle der nächsten Packung in gleicher Richtung fortsetzen.
Zweckmäßig ist weiter in solchen geordneten Kolonnenpackungen, daß der Durchmesser der Stege, Streifen o.ä. im Bereich von einigen mm liegt, während die Länge an sich nicht begrenzt ist, sofern ein genügend großer Winkel der Stege gegenüber der Vertikalen gewählt ist.
Zweckmäßig geht jedoch die Höhe der Teilpackungen oder Schichten nicht über etwa 20 cm hinaus.
Bei zylinderförmigen Teilpackungen kann zweckmäßig der Durchmesser zur Höhe 1 : 0,5 bis 1 : 3, besonders 1 : 1 bis 1 : 2,5 betragen.
Ganz wesentlich ist der Winkel der Hohlkanäle von etwa 30 bis etwa 60° gegenüber der Vertikalen.
Alle für die Kolonnenpackung verwendeten Materialien müssen bei Betriebstemperaturen bis zu 180°C beständig sein.
Da die Zersetzungstemperaturen der Einsatzstoffe bei 150-190°C liegen, dürfen diese Temperaturen weder in der Destillierblase noch in der Kolonne überschritten werden. Andererseits liegen die Siedepunkte, besonders der hochchlorierten Anteile bei 6 - 20 mbar in diesem Temperaturbereich.
Es ist daher überraschend, daß gemäß der vorliegenden Erfindung erstmals eine Vakuum-Rektifikation, d.h. eine Vakuum-Destillation bei Rücklauf einer gewählten Menge

Kopfkondensat in der Kolonne überhaupt möglich ist und daß jede Zersetzung bei der Rektifikation so völlig vermieden ist, daß die Vakuum-Rektifikation überhaupt und erstmals die Stofftrennung und Reindarstellung der einzelnen Stoffe mit Reinheiten von über 93, zumeist weit über 96 % möglich wurde.
Der Druck hat daher zwischen 1 und 120 mbar, bevorzugt zwischen 6 bis 30 mbar zu liegen.

Es war sehr überraschend, daß der Druckabfall je theoretischer Trenneinheit nicht wie bei konventionellen Trenneinheiten vom Kolonnendurchmesser abhängt.

Der Temperaturbereich zwischen Verdampfung, Rektifikation und Kondensation ist sehr gering, beispielsweise bei $\alpha,\alpha'$-Dichlor-p-Xylol (XVIII) nur knapp 50°C. Da andererseits zur Erreichung einer Produkteinheit von etwa 98 % eine Mindestzahl von theoretischen Trennstufen erforderlich sind, wobei letztere im Fall der Trennung von XVII und XVIII bei 22 liegt, ist mit einem erheblichen Temperaturgradienten über die Kolonnenlänge, hervorgerugen durch den Druckverlust der Kolonnenpackung mit der erforderlichen Trennleistung, zu rechnen.
Die hohe spezifische Trennleistung innerhalb eines schmalen Arbeitsbereichs der Temperatur zu erreichen, kann nur mit Kolonnenpackung von geringem Druckverlust pro theoretischer Trennstufe erreicht werden. Daher kommt der geringen Raumerfüllung, der geordneten sich in allen Raumrichtungen wiederholende Hohlstruktur der Kolonnenpackungen hohe Bedeutung zu, um Druckverluste von lediglich 0,1 bis 1 mbar je theoretischer Trennstufe zu erreichen.

Vergleichbare Betriebsbedingungen ergeben je theoretischer Trennstufe nach Winnacker/Küchler, Chemische Technologie,

Band 7, Carl Hanase Verlag, München 1975, Seite 199-203, bei Glocken- und Siebböden 2 bis 8 mbar und bei Füllkörperschüttungen (Raschig-Ringe, Pall-Ringe, Berl-Sättel) 1 bis 6 mbar, so daß durch zu hohe Temperaturgradienten im Falle der seitenkettenchlorierten Alkylaromomaten dann Zersetzung auftritt.

Die vorliegende Erfindung ist also eine Lösung einer sehr lange bestehenden Aufgabe, zudem mit Maßnahmen, deren Erfolg aus der Kenntnis des Standes der Technik nicht erwartet werden konnte.

Durch Anwendung geordneter Kolonnenpackungen ist es möglich, im technischen Rahmen besonders die Komponenten I bis XXXII aus ihren Gemischen durch Rektifikation in reiner Form zu erhalten.

Das keramische Material der Packung ist gegen die Arbeitsstoffe beständig - nach einigen hundert Betriebsstunden konnte kein Materialabtrag festgestellt werden - und führt auch zu keinerlei Zersetzung des Arbeitsstoffes selbst.

Ebenso wurde keine Verschmutzung des Packungsmaterials etwa durch Aufwachsungen usw. beobachtet.

Die Betriebsweise kann kontinuierlich oder diskontinuierlich gestaltet werden. Die erstere wird bevorzugt angewandt, da die Regelung der Kolonne einfacher ist.

Darüber hinaus läßt sich der Betriebsinhalt an Arbeitsstoff gegenüber dem Chargenbetrieb bedeutend geringer halten. Die Gewinnung einer Komponente als Reinstoff wird nach bekannten Verfahren im Chargenbetrieb durch Entnahme von Vorlauf, Hauptlauf ( gewünsches Produkt ) und Nachlauf am Kolonnenkopf, im kontinuierlichen Betrieb durch Entnahme der Stoffströme aus Kopf und Sumpf in mindestens zwei Druchgängen, wobei Vorlauf, Hauptlauf und ggf. Nachlauf nacheinander am Kopf abgenommen werden, oder durch gleichzeitige Entnahme von Vorlauf, Hauptlauf und ggf. Nachlauf an den Köpfen von mindestens zwei Kolonnen vorgenommen.

Sollen mehrere Komponenten rein gewonnen werden, können dementsprechend mehrere Hauptläufe abgenommen werden.
Die Produktreinheiten liegen je nach Schwierigkeitsgrad der Trennung, für den die relative Flüchtigkeit ein Maß ist, zwischen 90 und 99,9 %, die Ausbeute, angegeben als Anteil der gewonnenen an der insgesamt vorhandenen Produktmenge, liegen zwischen 85 und 98 % des vorhandenen Materials.
Vorlauf und Nachlauf, letzterer ggf. nach vorangehender Reinigung durch Flash-Destillation, können durch weitergehende Chlorierung mit elementarem Chlor unter Lichtkatalyse gemäß dem anfangs beschriebenen Verfahren in höher seitenkettenchlorierte Gemische von Abkömmlingen der Xylole oder Chlortoluole oder auch in die vollständig oder nahezu vollständig in der Seitenkette chlorierten Verbindungen VII, XV, XXIII, XXVI, XXIX und XXXII überführt werden.
Die Reinheiten und Ausbeuten aus erneut chlorierten Stof fen liegen nicht unter denjenigen Werten, die durch einmalige Chlorierung der Xylole und Chlortoluole zu den genannten Produkten erzielt werden.
Somit sind, ebenfalls erstmalig, nicht verwendbare Nebenprodukte des Rektifikationsprozesses durch Überführung in verwendbare Produkte einer sinnvollen Verwertung zugeführt.
Nebenprodukte, zu verwertende Hilfsstoffe oder unbrauchbare Stoffreste fallen bei dem vorliegenden Verfahren nicht an. Die Menge des Rückstands ist infolge der ver miedenen Zersetzung außerordentlich klein.
Erstmals werden technisch bzw. überhaupt eine Anzahl von Stoffen aus den Chlorierungsgemischen in reiner Form bzw. in bisher unbekannter Reinheit zugängig.
Die Reinheit der Produkte kann durch die Höhe der Kolonne d.h. die Zahl der theoretischen Böden, durch Wiederholung der Rektifikation mit bestimmten Fraktionen mit hohen Gehalten des Zielprodukts und weitere übliche Maßnahmen gesteigert werden. Die in den Beispielen angegebenen Reinheiten der Hauptfraktionen dürfen daher nicht als verfahrensgemäß erreichbare Höchstwerte der Reinheit verstanden werden.

Bei den Trennelementen A des Beispiels 1 und den Trennelementen B des Beispiels 7 handelt es sich um übereinander gelegte zylindrische Teilpackungen mit einem Verhältnis von Durchmesser zu Höhe von etwa 1 : 1,2. Das keramische Material enthält u.a. etwa 18 Gew.-% $ZrO_2$ und etwa 79 Gew.-% $SiO_2$. Die Zylinder bestehen aus vertikal nebeneinander liegenden Schichten aus in der Schicht aufeinanderfolgenden, zueinander parallelen Wellen ( etwa in der Art von Wellblech ), jedoch verlaufen die Wellen etwa mit $25^o$ Neigung gegen die Vertikale, worauf eine gleichartige wellenförmige Schicht mit gleicher Neigung gegen die Vertikale, jedoch in der anderen Richtung folgt.

Die im gleichen Winkel geneigten Kanäle haben den Querschnitt eines Dreiecks mit stark abgerundeten Ecken. Alle Berührungsstellen sind durch aufgestäubte Keramikkörnung fest aneinander gesintert. Bei der Kolonnenpackung von der Nennweite 30 mm, gibt es 6 bis 8 geneigte Kanäle in Sicht des Zylinderdurchmessers, bei der Packung der Nennweite 70 mm ( Beispiel 7 ) entsprechend mehr.

Dr.La/Ra

Beispiel 1

5,58 kg eines Gemisches, das durch Seitenkettenchlorierung von 3,34 kg o-Xylol erhalten wurde, mit der folgenden Zusammensetzung:

| | |
|---|---|
| α-Chlor-o-Xylol (I) | 16,14 Gew.-% |
| α,α-Dichlor-o-Xylol (V) | 7,68 " |
| α,α'-Dichlor-o-Xylol (II) | 54,00 " |
| α,α,α'-Trichlor-o-Xylol (III) | 18,32 " |
| α,α,α',α'-Tetrachlor-o-Xylol (VI) | 1,49 " |
| Andere Komponenten | 2,37 " |

werden in der nachfolgend beschriebenen Rektifizierkolonne chargenweise aufgetrennt.

Auf eine heizbare Destillationsblase von 7 l Inhalt ist eine beheizbare Vakuum-Fraktionierkolonne von 1 m Höhe und 30 mm Durchmesser aufgesetzt, deren Innenraum mit den zylinderförmigen Trennelementen A von 16 cm Höhe ausgestattet ist, die übereinander liegend den gesamten Innenraum der Kolonne einnehmen und 6 bis 8 theoretischen Trennstufen (Böden) entsprechen. Oben schließt sich ein mit Thermostat betriebener Dephlegmator mit Rücklaufteiler an. Zur Aufnahme des am Kopf ablaufenden Produkts ist eine Vorlage mit Wechseleinrichtung für Vakuumbetrieb vorhanden.

Bei einem Kopfdruck von 20 mbar, einer Gasbelastung von 2,15 kg/h und einem Rücklaufverhältnis von 10 : 1 steigt die Kopftemperatur während des Rektifikationsvorganges von 86 auf 123°C, die Temperatur des Blaseninhalts von 125 auf 141°C allmählich an.

Die Druckdifferenz zwischen Kopf und Blase liegt unterhalb 1 mbar. Die Trennergebnisse sind in der folgenden Aufstellung zusammengefaßt:

| Komponente | Zusammensetzung [Gew.-%] Vorlauf | Hauptlauf | Nachlauf |
|---|---|---|---|
| I | 56,20 | - | - |
| V | 28,47 | 1,08 | 0,19 |
| II | 14,88 | 97,47 | 10,98 |
| III | 0,07 | 1,09 | 81,46 |
| IV | - | - | 2,41 |
| andere | 0,38 | 0,36 | 4,96 |
| Mengen [kg] | 1,52 | 2,69 | 1,23 |

Die erhaltenen 2,69 kg an II vom Schmelzpunkt 55°C entsprechen einer Rektifikationsausbeute von 87 %. Der Rückstand von 0,07 kg, (1,25 Gew.-%) ist gering. Zersetzungserscheinungen unter Abspaltung von Chlorwasserstoff werden nicht beobachtet, desgleichen keine Belegungen der keramischen Trennelemente. Vorlauf und Nachlauf ergeben, getrennt oder gemeinsam erschöpfend mit Chlor unter den Bedingungen der Seitenkettenchlorierung behandelt, 4,28 kg α,α,α,α',α'-Pentachlor-o-Xylol (VII) einer Reinheit von 98,7 % nach gaschromatographsicher Analyse. Der Vorlauf kann auch auf den gleichen Chlorierungsgrad wie das ursprüngliche Chlorierungsgemisch chloriert werden und ergibt dann bei gleichartiger Fraktionierung die angeführten Ergebnisse.

Beispiel 2

6,53 kg eines Gemisches, das durch Seitenkettenchlorierung von 2,84 kg o-Xylol erhalten wurde, mit der folgenden Zusammensetzung

| | |
|---|---|
| α,α'-Dichlor-o-Xylol (II) | 1,76 Gew.-% |
| α,α,α'-Trichlor-o-Xylol (III) | 29,34 " |
| α,α,α,α'-Tetrachlor-o-Xylol (IV) | 3,22 " |
| α,α,α',α'-Tetrachlor-o-Xylol (VI) | 56,14 " |

| | | |
|---|---|---|
| α,α,α,α',α'-Pentachlor-o-Xylol (VII) | 8,39 | Gew.-% |
| Andere Komponenten | 1,15 | " |

werden in der in Beispiel 1 beschriebenen Apparatur der Rektifikation unterworfen. Kopfdruck: 8 mbar. Gasbelastung: 2,1 kg/h. Rücklaufverhältnis 10 : 1. Die Kopftemperatur steigt von 106 auf 141°C und die Temperatur des Blaseninhalts von 133 auf 158°C allmählich an. Die Druckdifferenz zwischen Kopf und Blase der Kolonne liegt unterhalb 1 mbar. Trennergebnisse:

| Komponente | Zusammensetzung [Gew.-%] Vorlauf | Hauptlauf | Nachlauf |
|---|---|---|---|
| II | 3,42 | - | - |
| III | 83,62 | 2,05 | - |
| IV | 1,52 | 1,30 | 0,25 |
| VI | 10,90 | 93,24 | 18,85 |
| VII | 0,19 | 3,27 | 75,33 |
| andere | 0,35 | 0,14 | 5,57 |
| Mengen [kg] | 2,29 | 3,46 | 0,73 |

Die erhaltenen 3,46 kg an VI vom Schmelzpunkt 90°C entsprechen einer Rektifikationsausbeute von 88 %. Der Rückstand beträgt 86 g (1,32 Gew.-%). Vorlauf und Nachlauf können wie in Beispiel 1 behandelt werden.

Beispiel 3

6,04 kg eines Gemisches, das durch Seitenkettenchlorierung von 3,83 kg m-Xylol erhalten wurde, mit der folgenden Zusammensetzung:

| | | |
|---|---|---|
| α-Chlor-m-Xylol (VIII) | 17,05 | Gew.-% |
| α,α-Dichlor-m-Xylol (XII) | 9,86 | " |
| α,α'-Dichlor-m-Xylol (IX) | 53,56 | " |
| α,α,α'-Trichlor-m-Xylol (X) | 14,20 | " |
| Andere Komponenten | 5,33 | " |

werden wie in Beispiel 1 beschrieben dem Rektifizierprozeß unterworfen. Kopfdruck: 15 mbar. Gasbelastung: 2,31 kg/h. Rücklaufverhältnis 10 : 1. Die Kopftemperatur steigt von 80 auf 141°C und die Temperatur des Blaseninhalts von 138 auf 163°C allmählich an. Die Druckdifferenz zwischen Kopf und Blase liegt unterhalb von 1 mbar.
Trennergebnisse:

| Komponente | Zusammensetzung [Gew.-%] Vorlauf | Hauptlauf | Nachlauf |
|---|---|---|---|
| VIII | 54,81 | - | - |
| XII | 28,34 | 0,06 | - |
| IX | 8,73 | 96,28 | 22,03 |
| X | - | 2,65 | 76,01 |
| andere | 8,12 | 1,01 | 1,96 |
| Mengen [kg] | 1,87 | 2,88 | 1,12 |

Die erhaltenen 2,88 kg an IX vom Schmelzpunkt 34°C entsprechen einer Rektifikationsausbeute von 85,6 %. Der Rückstand 65 g ( 1,08 Gew.-%). Vorlauf und Nachlauf ergeben, getrennt oder gemeinsam erschöpfend mit Chlor unter den Bedingungen der Seitenkettenchlorierung behandelt, 5,36 kg α,α,α,α',α',α'-Hexachlor-m-Xylol (XV) einer Reinheit von 97,8 % nach gaschromatographischer Analyse.

Beispiel 4

6,48 kg eines Gemisches, das durch Seitenkettenchlorierung von 2,62 kg p-Xylol erhalten wurde, mit der folgenden Zusammensetzung:

| | |
|---|---|
| α,α,α'-Trichlor-p-Xylol (XVIII) | 12,94 Gew.-% |
| α,α,α,α'-Tetrachlor-p-Xylol (XIX) | 4,94 " |
| α,α,α',α'-Tetrachlor-p-Xylol (XXI) | 50,04 " |
| α,α,α,α',α'-Pentachlor-p-Xylol (XXII) | 25,97 " |

| | |
|---|---|
| α,α,α,α',α',α'-Hexachlor-p-Xylol (XXIII) | 5,55 Gew.-% |
| Andere Komponenten | 0,56 " |

werden wie in Beispiel 1 beschrieben der Rektifikation unterworfen. Kopfdruck: 8 mbar. Gasbelastung 2,26 kg/h. Rücklaufverhältnis: 10 : 1. Die Kopftemperatur steigt von 129 auf 151°C und die Temperatur des Blaseninhalts von 147 auf 164°C allmählich an. Trennergebnisse:

| Komponente | Zusammensetzung [Gew.-%] Vorlauf | Hauptlauf | Nachlauf |
|---|---|---|---|
| XVIII | 70,32 | 1,56 | 0,51 |
| XIX | 21,52 | 2,26 | 0,57 |
| XXI | 8,12 | 92,52 | 15,80 |
| XXII | - | 3,26 | 67,03 |
| XXIII | - | 0,14 | 14,98 |
| andere | 0,04 | 0,26 | 1,11 |
| Mengen [kg] | 1,09 | 3,01 | 2,36 |

Die erhaltenen 3,01 kg an XXI vom Schmelzpunkt 93 - 95°C entsprechen einer Rektifikationsausbeute von 86 %. Rückstand: 102 g (1,58 Gew.-%). Vor- und Nachlauf ergeben nach Seitenkettenchlorierung mit Chlor 3,59 kg α,α,α,α',α',α'-Hexachlor-p-Xylol (XXIII) einer Reinheit von 99,63 % nach gaschromatographischer Analyse.

Beispiel 5

4,57 kg eines Gemisches, das durch Seitenkettenchlorierung von 3,60 kg p-Chlortoluol erhalten wurde, mit der folgenden Zusammensetzung:

| | |
|---|---|
| p-Chlortoluol | 14,01 Gew.-% |
| p-Chlorbenzylchlorid (XXX) | 68,35 " |
| p-Chlorbenzalchlorid (XXXI) | 15,17 " |
| Andere Komponenten | 2,47 " |

werden wie in Beispiel 1 beschrieben der Rektifikation unterworfen. Kopfdruck: 30 mbar. Gasbelastung: 1,95 kg/h. Rücklaufverhältnis 10 : 1. Die Kopftemperatur steigt von 58 auf 126°C und die Temperatur des Blaseninhalts von 124 auf 140°C während des Rektifizierens an. Die Druckdifferenz zwischen Kopf und Blase liegt unterhalb 1 mbar. Trennergebnisse:

| Komponente | Zusammensetzung [Gew.-%] Vorlauf | Hauptlauf | Nachlauf |
|---|---|---|---|
| p-Chlortoluol | 98,32 | 1,75 | - |
| XXX | 1,68 | 95,32 | 2,81 |
| XXXI | - | 2,53 | 87,37 |
| andere | - | 0,40 | 9,82 |
| Mengen [kg] | 0,66 | 2,98 | 0,81 |

Die 2,98 kg an XXX vom Schmelzpunkt 29°C entsprechen einer Rektifikationsausbeute von 91 %. Rückstand: 82 g (1,81 Gew.-%) des Einsatzes. Zersetzungserscheinungen treten nicht auf. Vorlauf und Nachlauf ergeben, getrennt oder gemeinsam durch erschöpfende Chlorierung der Seitenketten 2,01 kg p-Chlorbenzotrichlorid (XXXII) einer Reinheit von 98,4 % nach gaschromatographischer Analyse.

Beispiel 6

4,36 kg eines Gemisches, das durch Seitenkettenchlorierung von 2,71 kg o-Chlortoluol erhalten wurde, mit der folgenden Zusammensetzung

| | | |
|---|---|---|
| o-Chlortoluol | 0,88 | Gew.-% |
| o-Chlorbenzylchlorid (XXIV) | 20,42 | " |
| o-Chlorbenzalchlorid (XXV) | 67,93 | " |
| o-Chlorbenzotrichlorid (XXVI) | 8,90 | " |
| Andere Komponenten | 1,87 | " |

werden wie in Beispiel 1 beschrieben der Rektifikation unterworfen. Kopfdruck: 30 mbar. Gasbelastung: 1,98 kg/h. Rücklaufverhältnis: 10 : 1. Die Kopftemperatur steigt von 102 auf 139°C, die Temperatur des Blaseninhalts von 131 auf 152°C während des Rektifizierens an.
Die Druckdifferenz zwischen Kopf und Blase liegt unterhalb 1 mbar. Trennergebnisse:

| Komponente | Zusammensetzung [Gew.-%] Vorlauf | Hauptlauf | Nachlauf |
|---|---|---|---|
| o-Chlortoluol | 3,34 | - | - |
| XXIV | 86,71 | 0,80 | - |
| XXV | 8,22 | 97,28 | 18,21 |
| XXVI | - | 1,72 | 74,22 |
| andere | 1,73 | 0,20 | 7,57 |
| Mengen [kg] | 1,02 | 2,78 | 0,48 |

Die 2,78 kg an XXV vom Schmelzpunkt < 30°C entsprechen einer Rektifikationsausbeute von 91,5 %. Rückstand 53 g (1,22 Gew.-%). Zersetzungserscheinungen treten nicht auf. Vor- und Nachlauf ergeben, getrennt oder gemeinsam durch erschöpfende Chlorierung der Seitenketten 1,86 kg o-Chlorbenzotrichlorid (XXVI) einer Reinheit von 96,2 % nach gaschromatographischer Analyse.

Beispiel 7

418 kg eines Gemisches, das durch Chlorierung von 247 kg p-Xylol erhalten wurde, mit der folgenden Zusammensetzung

| | | |
|---|---|---|
| α-Chlor-p-Xylol (XVI) | 7,45 | Gew.-% |
| α,α-Dichlor-p-Xylol (XX) | 8,72 | " |
| α,α'-Dichlor-p-Xylol (XVII) | 52,21 | " |
| α,α,α'-Trichlor-p-Xylol (XVIII) | 25,85 | " |
| α,α,α,α'-Tetrachlor-p-Xylol (XIX) | 0,75 | " |
| α,α,α',α'-Tetrachlor-p-Xylol (XXI) | 3,50 | " |
| Andere Komponenten | 1,52 | " |

werden in einer heizbaren Rektifizierkolonne getrennt, die der des Beispiels 1 im wesentlichen entspricht, jedoch 70 mm Durchmesser und eine Trennlänge von 2,30 m aufweist und mit den zylinderförmigen keramischen Trennelementen B von 16 cm Höhe randschlüssig gefüllt ist. Durch einen Zulauf auf halber Höhe ist die Kolonne für den kontinuierlichen Betrieb ausgerüstet. Zur Gewinnung der Komponente XVII als reinen Stoff wird dreimal wie folgt rektifiziert. Der erste Durchgang wird bei einem Kopfdruck von 18 mbar und einem Rücklaufverhältnis von 2,5 : 1, der zweite Durchgang bei einem Kopfdruck von 10 mbar und einem Rücklaufverhältnis von 2,3 : 1, der dritte Durchgang bei einem Kopfdruck von 10 mbar ohne Rücklauf ausgeführt. Die Gasbelastung beträgt in allen Durchgängen 5,4 kg/h. Die Destillate der Durchgänge werden getrennt aufgefangen. Die Kopftemperaturen betragen in der Reihenfolge der Durchgänge 93, 130 und 136°C, die Temperaturen des Verdampferinhalts 143, 155 und 151°C. Die Druckdifferenz zwischen Kopf und Verdampfer liegt im Bereich um 1 mbar. Trennergebnisse:

| Komponente | Zusammensetzung des Destillats [Gew.-%] Durchgang 1 | 2 | 3 |
| --- | --- | --- | --- |
| XVI | 41,83 | 0,07 | - |
| XX | 48,76 | 0,26 | - |
| XVII | 9,24 | 98,57 | 2,23 |
| XVIII | - | 0,53 | 83,56 |
| XIX | - | 0,07 | 2,09 |
| XXI | - | 0,08 | 10,16 |
| andere | 0,17 | 0,42 | 1,96 |
| Mengen [kg] | 65,8 | 211,3 | 138,0 |

Die 211,3 kg an XVII vom Schmelzpunkt 100 - 101°C entsprechen einer Rektifikationsausbeute von 95,4 %. Der Rückstand beträgt 2,6 kg (0,62 Gew.-%). Zersetzungserscheinungen treten nicht auf. Vor- und Nachlauf ergeben, getrennt oder gemeinsam erschöpfend mit Chlor unter den Bedingungen der Seitenkettenchlorierung behandelt, $\alpha,\alpha,\alpha,\alpha',\alpha',\alpha'$-Hexachlor-p-Xylol einer Reinheit von 99,42 %, oder bei teilweiser Chlorierung, Gemische entsprechend den Einsatzstoffen der Beispiele 4 und 8, welche nach gaschromatographischer Analyse durch erneute Rektifikation einen oder mehrere gewünschte Reinstoffe liefern.

Beispiel 8

328 kg eines Gemisches, das durch Chlorierung von 132 kg p-Xylol erhalten wurde, mit der Zusammensetzung

| | |
|---|---|
| $\alpha,\alpha,\alpha'$-Trichlor-p-Xylol (XVIII) | 12,94 Gew.-% |
| $\alpha,\alpha,\alpha,\alpha'$-Tetrachlor-p-Xylol (XIX) | 4,94 " |
| $\alpha,\alpha,\alpha',\alpha'$-Tetrachlor-p-Xylol (XXI) | 50,04 " |
| $\alpha,\alpha,\alpha,\alpha',\alpha'$-Pentachlor-p-Xylol (XXII) | 25,97 " |
| $\alpha,\alpha,\alpha,\alpha',\alpha',\alpha'$-Hexachlor-p-Xylol (XXIII) | 5,55 " |
| Andere Komponenten | 0,56 " |

werden wie in Beispiel 7 beschrieben der Rektifikation unterworfen um Komponente XXI als Reinstoff zu gewinnen. Der erste Durchgang wird bei einem Kopfdruck von 10 mbar und einem Rücklaufverhältnis von 13 : 1, der zweite Durchgang bei einem Kopfdruck von 8 mbar und einem Rücklaufverhältnis von 9 : 1, der dritte Druchgang ohne Rücklauf bei einem Kopfdruck von 8 mbar durchgeführt. Die Gasbelastung beträgt in allen Durchgängen 5,4 kg/h. Die Kopftemperaturen betragen, in der Reihenfolge der Durchgänge, 143, 144 und 150°C, die Temperaturen des Verdampferinhalts 161, 163 und 168°C. Die Druckdifferenz zwischen Kopf und Verdampfer

liegt im Bereich um 1 mbar. Trennergebnisse:

| Komponente | Zusammensetzung des Destillats [Gew.-%] Durchgang 1 | 2 | 3 |
|---|---|---|---|
| XVIII | 63,07 | 0,41 | - |
| XIX | 22,80 | 1,08 | - |
| XXI | 12,87 | 96,23 | 7,74 |
| XXII | 1,17 | 2,14 | 74,68 |
| XXIII | - | - | 15,85 |
| andere | 0,09 | 0,14 | 1,73 |
| Mengen [kg] | 62,8 | 151,6 | 111,3 |

Die 151,6 kg an XXI vom Schmelzpunkt 94 - 96°C entsprechen einer Rektifikationsausbeute von 89 %. Rückstand 1,93 kg (0,59 Gew.-%) des Einsatzes. Vor- und Nachlauf ergeben, getrennt oder gemeinsam mit Chlor unter den Bedingungen der Seitenkettenchlorierung behandelt, α,α,α,α',α',α'-Hexachlor-p-Xylol (XXIII) einer Reinheit von 99,13 % nach gaschromatographischer Analyse.

Dr.La/Ra

Patentansprüche

1. Verfahren zur Reindarstellung seitenkettenchlorierter Alkylaromaten durch Destillation, dadurch gekennzeichnet, daß Gemische beliebiger Zusammensetzung mit Chlorgehalten zwischen 4 und 65 Gew.-% Chlor in der Seitenkette, hergestellt durch Seitenkettenchlorierung von Alkylaromaten oder deren im Kern chlorierten Derivaten, durch fraktionierte Destillation bei Sumpftemperaturen von 100 bis 180°C und vermindertem Druck von 1 bis 120 mbar, bevorzugt von 6 bis 30 mbar, in die einzelnen Komponenten aufgetrennt werden, wobei Füllkörper-Kolonnen mit aus Glas oder keramischem Material bestehende Kolonnenpackungen Verwendung finden, deren Druckverlust pro theoretischer Trennstufe weniger als ein mbar im gesamten Betriebsbereich beträgt und Spuren von Schwermetallen ausgeschlossen sind.

2. Verfahren nach Anspruch 1, dadurch gekennzeichnet, daß die Kolonnenpackungen eine geordnete, sich stetig in allen Raumrichtungen wiederholende Hohlstruktur aufweisen.

3. Verfahren nach einem der Ansprüche 1 oder 2, dadurch gekennzeichnet, daß die Raumerfüllung der Kolonnenpackung 10 - 30 % beträgt.

4. Verfahren nach den Ansprüchen 1 bis 3, dadurch gekennzeichnet, daß neben der Hauptfraktion anfallende Nebenfraktionen entweder in wieder verwendbare Destillationsgemische oder vollständig in der Seitenkette chlorierte Verbindungen durch Behandlung mit Chlor überführt werden.

Dr.La/Ra

0054634

Nummer der Anmeldung

EP 81 10 796

Europäisches Patentamt

# EUROPÄISCHER RECHERCHENBERICHT

| EINSCHLÄGIGE DOKUMENTE | | | KLASSIFIKATION DER ANMELDUNG (Int Cl.) |
|---|---|---|---|
| Kategorie | Kennzeichnung des Dokuments mit Angabe, soweit erforderlich, der maßgeblichen Teile | betrifft Anspruch | |
| A,D | GB - A - 1 410 474 (ALBRIGHT AND WILSON) | | C 07 C 21/24<br>C 07 C 25/00<br>C 07 C 17/38 |
| A | US - A - 2 851 498 (R.R.DREISBACH et al.) | | |
| | ---------- | | |

RECHERCHIERTE SACHGEBIETE (Int Cl.)

C 07 C 17/00
21/00
25/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| DEN HAAG | 18-03-1982 | VAN GEYT |